# EUROPEAN PATENT APPLICATION

(11) **EP 3 590 491 A1**
(43) Date of publication of application: **08.01.2020**
(21) Application number: 18760469.9
(22) Date of filing: 28.02.2018
(51) Int. Cl.: A61K 8/06, A61K 8/34, A61K 8/37, A61K 8/44, A61K 8/49, A61Q 1/12, A61Q 17/04

(54) **OIL-IN-WATER EMULSION COSMETIC COMPOSITION**

(30) Priority: 02.03.2017 JP 2017039484
(71) Applicant: Shiseido Company Ltd., Chuo-ku Tokyo 104-0061 (JP)
(72) Inventor: OOTAKE, Sawako, Yokohama-shi Kanagawa 224-8558 (JP); YABU, Momo, Yokohama-shi Kanagawa 224-8558 (JP)
(74) Representative: Ter Meer Steinmeister & Partner
(86) International application number: PCT/JP2018/007521
(87) International publication number: WO 2018/159683

(57) **Abstract**

The purpose of the present invention is to provide an oil-in-water emulsion cosmetic composition which has high ultraviolet light blocking ability without being blended with ethylhexyl methoxycinnamate and octocrylene that are liquid ultraviolet absorbents, and which achieves excellent feeling of use by suppressing the blending amount of a polar oil. The present invention relates to an oil-in-water emulsion cosmetic composition which contains (A) an ultraviolet absorbent that contains (a1) a sulfonic acid group-containing water-soluble ultraviolet absorbent, (a2) bisethyloxyphenol methoxyphenyl triazine, (a3) diethylamino hydroxybenzoyl hexyl benzoate and (a4) ethylhexyl triazone, (B) a polar oil, (C) a higher alcohol and (D) a nonionic surfactant, and wherein the total blending amount of (a2) bisethyloxyphenol methoxyphenyl triazine, (a3) diethylamino hydroxybenzoyl hexyl benzoate, (a4) ethylhexyl triazone and (B) the polar oil is 45% by mass or less.

## Description

### TECHNICAL FIELD

The present invention relates to an oil-in-water emulsion cosmetic. More specifically, the present invention relates to an oil-in-water emulsion cosmetic that has excellent emulsion stability and that exhibits high ultraviolet ray protection effects, even while having a low oil content and without containing ethylhexyl methoxycinnamate and octocrylene, which are liquid ultraviolet ray absorbing agents that have conventionally been commonly used.

### BACKGROUND ART

Oil-in-water emulsion cosmetics that exhibit ultraviolet ray protection effects such as, for example, sunscreen cosmetics, have conventionally blended an appropriate combination of oil-based ultraviolet ray absorbing agents and powder-type ultraviolet ray scattering agents in order to effectively screen ultraviolet rays spanning from the UVA range to the UVB range. However, there were problems in that, if too much of an oil-based ultraviolet ray absorbing agent is blended, a sticky texture is imparted, and if the blended amount of the ultraviolet ray scattering agent is too high, there is a powdery feeling in use, and the skin becomes unnaturally white.

Patent Document 1 describes that, by blending four specific types of ultraviolet ray absorbing agents, a high SPF (Sun Protection Factor) was able to be achieved even when not blending or when suppressing the blended amount of ultraviolet ray scattering agents.

Meanwhile, it has been shown that octyl methoxycinnamate (ethylhexyl methoxycinnamate) and octocrylene, which are commonly used as ultraviolet ray absorbing agents, have irritant properties. Thus, a sunscreen cosmetic in which, instead of the above-mentioned substances, a specific ultraviolet ray scattering agent (rutile crystalline titanium oxide or the like) is blended in order to ensure that ultraviolet ray protection effects are obtained, has also been proposed (Patent Document 2).

However, the aforementioned liquid ultraviolet ray absorbing agents not only have ultraviolet ray absorbing properties in themselves, but they also function as good solvents for solid or poorly soluble ultraviolet ray absorbing agents that are co-blended therewith (see, for example, Patent Document 3). Therefore, when these liquid ultraviolet ray absorbing agents are not blended, it is necessary to increase the amount of polar oils that serve as solvents in order to prevent the precipitation or destabilization of other ultraviolet ray absorbing agents, and as a result thereof, there was a problem in that stickiness occurred.

### RELATED ART

### PATENT DOCUMENTS

Patent Document 1: WO 2010/110020
Patent Document 2: JP 2015-124172 A
Patent Document 3: JP 2014-240382 A

### SUMMARY OF THE INVENTION

### PROBLEM TO BE SOLVED BY THE INVENTION

Thus, the problem addressed by the present invention is that of providing an oil-in-water emulsion cosmetic that not only provides high ultraviolet protection properties, but that also has an excellent feeling in use and emulsion stability, even without blending ethylhexyl methoxycinnamate and octocrylene, which are liquid ultraviolet ray absorbing agents, and also suppressing the blended amount of polar oils.

### MEANS FOR SOLVING THE PROBLEM

As a result of performing diligent investigations towards solving the above-mentioned problems, the present inventors discovered that an oil-in-water emulsion cosmetic that exhibits high ultraviolet ray protection properties while also having excellent stability and usability is obtained by blending, as ultraviolet ray absorbing agents, a combination of specific water-soluble absorbing agents and oil-based solid absorbing agents, and further introducing an α-gel structure to the emulsion system.

In other words, the present invention provides an oil-in-water emulsion cosmetic characterized by containing:
(A) an ultraviolet ray absorbing agent containing
   (a1) a sulfonic acid group-containing water-soluble ultraviolet ray absorbing agent,
   (a2) bis-ethyloxyphenol methoxyphenyl triazine,
   (a3) diethylamino hydroxybenzoyl hexyl benzoate, and
   (a4) ethylhexyl triazone;
(B) a polar oil;
(C) a higher alcohol; and
(D) a non-ionic surfactant; wherein a total blended amount of the (a2) bis-ethyloxyphenol methoxyphenyl triazine, the (a3) diethylamino hydroxybenzoyl hexyl benzoate, the (a4) ethylhexyl triazone, and the (B) polar oil is 45 mass% or less.

### EFFECTS OF THE INVENTION

The oil-in-water emulsion cosmetic of the present invention has excellent emulsion stability, exhibits high ultraviolet ray protection effects, and also lacks irritation and has a good texture when used, even when ethylhexyl methoxycinnamate and octocrylene, which are liquid ultraviolet ray absorbing agents, are not blended, and the blended amount of polar oils is also suppressed.

### MODES FOR CARRYING OUT THE INVENTION

The oil-in-water emulsion cosmetic (hereinafter also referred to simply as "emulsion cosmetic") of the present invention contains an ultraviolet ray absorbing agent (component A). The ultraviolet ray absorbing agent of the present invention contains, as essential components, a sulfonic acid group-containing water-soluble ultraviolet ray absorbing agent (component a1), bis-ethyloxyphenol methoxyphenyl triazine (component a2), diethylamino hydroxybenzoyl hexyl benzoate (component a3), and ethylhexyl triazone (component a4).

The (a1) sulfonic acid group-containing water-soluble ultraviolet ray absorbing agent is a water-soluble ultraviolet ray absorbing agent having a sulfonic acid group in the molecule.

The component (a1) is preferably, but not limited to being, a benzylidene camphor derivative or a phenylbenzimidazole derivative having a sulfonic acid group, or the like.

Specific examples of phenylbenzimidazole derivatives include phenylbenzimidazole sulfonic acid (labeling name) and the like, and the product that is commercially available from Merck under the product name Eusolex 232 can be favorably used.

Examples of benzylidene camphor derivatives include benzylidene camphor sulfonic acid and terephthalylidene dicamphor sulfonic acid and the like, both of which are commercially available from Chimex under the product names Mexoryl SD and Mexoryl SX.

The blended amount of the sulfonic acid group-containing water-soluble ultraviolet ray absorbing agent (component a1) in the emulsion cosmetic of the present invention is preferably 0.1 to 10 mass%, more preferably 0.3 to 8 mass%, and even more preferably 0.5 to 5 mass%.

The (a2) bis-ethyloxyphenol methoxyphenyl triazine (labeling name) is an oil-based solid ultraviolet ray absorbing agent that is commercially available from BASF under the product name Tinosorb S.

The blended amount of the bis-ethyloxyphenol methoxyphenyl triazine (component a2) in the emulsion cosmetic of the present invention is preferably 0.1 to 10 mass%, more preferably 0.3 to 8 mass%, and even more preferably 0.5 to 5 mass%.

The (a3) diethylamino hydroxybenzoyl hexyl benzoate (labeling name) is an oil-based viscous liquid or solid ultraviolet ray absorbing agent that is commercially available from BASF under the product name Uvinul A Plus.

The blended amount of the diethylamino hydroxybenzoyl hexyl benzoate (component a3) in the emulsion cosmetic of the present invention is preferably 0.1 to 10 mass%, more preferably 0.3 to 8 mass%, and even more preferably 0.5 to 5 mass%.

The (a4) ethylhexyl triazone (labeling name) is an oil-based solid ultraviolet ray absorbing agent that is commercially available from BASF under the product name Uvinul T-150.

The blended amount of the ethylhexyl triazone (component a4) in the emulsion cosmetic of the present invention is preferably 0.1 to 10 mass%, more preferably 0.3 to 8 mass%, and even more preferably 0.5 to 5 mass%.

The total blended amount of the components (a1), (a2), (a3) and (a4) is preferably 1.0 to 20 mass%, more preferably 1.5 to 19 mass%, and even more preferably 2.0 to 18 mass%. Sufficient ultraviolet ray protection effects can be obtained by adjusting the total blended amount within the aforementioned ranges.

The ultraviolet ray absorbing agent in the emulsion cosmetic of the present invention may be composed of only the above-mentioned ultraviolet ray absorbing agents (a1) to (a4), but in addition thereto, it is possible to blend other water-soluble or oil-soluble ultraviolet ray absorbing agents within a range not inhibiting the effects of the present invention. Examples of other ultraviolet ray absorbing agents include benzoic acid derivatives, salicylic acid derivatives, cinnamic acid derivatives, dibenzoylmethane derivatives, β,β-diphenyl acrylate derivatives, benzophenone derivatives, benzylidene camphor derivatives, phenylbenzimidazole derivatives, triazine derivatives, phenylbenzotriazole derivatives, anthranil derivatives, imidazoline derivatives, benzalmalonate derivatives, 4,4-diaryl butadiene derivatives and the like. However, ethylhexyl methoxycinnamate and octocrylene are not to be blended therein.

The total blended amount of the oil-soluble ultraviolet ray absorbing agents including (a2), (a3) and (a4) is preferably 0.5 to 19.5 mass%, more preferably 0.8 to 17 mass%, and even more preferably 1 to 15 mass%.

The polar oil (component B) that is blended in the emulsion cosmetic of the present invention simply needs to be a polar oil that is normally blended into cosmetics or the like, and is not particularly limited. Specifically, an ester oil, particularly an ester oil having an IOB value of approximately 0.1 to 0.5, is preferably used.

Specific examples of the polar oil (component B) include, but are not limited to, diisopropyl sebacate, pentaerythrityl tetraethylhexanoate, cetyl ethylhexanoate, jojoba oil, di(phytosteryl/octyldodecyl) lauroyl glutamate, triisostearin, glyceryl diisostearate, triethylhexanoin, (phytosteryl/behenyl) dimer dilinoleate, (phystosteryl/isostearyl/cetyl/stearyl/isostearyl/cetyl/stearyl/behenyl) dimer dilinoleate, isopropyl palmitate, phytosteryl macadamiate, pentaerythrityl tetra(behenate/benzoate/ethylhexanoate), ethylhexyl palmitate, myristyl myristate, isopropyl myristate, tripropylene glycol dipivalate, isodecyl neopentanoate and the like. Furthermore, a liquid ultraviolet ray absorbing agent may also be included in the polar oil (component B). Therefore, in the present invention, when calculating the total blended amount of the both component A and component B, liquid ultraviolet ray absorbing agents that are oil-soluble and polar are also included among the polar oils (component B).

The blended amount of the polar oil (component B) in the emulsion cosmetic of the present invention must be set so that the total blended amount of (a2) bis-ethyloxyphenol methoxyphenyl triazine, (a3) diethylamino hydroxybenzoyl hexyl benzoate, (a4) ethylhexyl triazone and the (B) polar oil is 45 mass% or lower. If this total blended amount exceeds 45 mass%, then a stable emulsion cannot be obtained. Needless to say, 45 mass% or lower includes, for example, all of the following values: 44 mass%, 43 mass%, 42 mass%, 41 mass%, 40 mass%, 39 mass% or lower, 38 mass% or lower, 37 mass% or lower, 36 mass% or lower, 35 mass% or lower, and the like. Though there is no particular restriction on the lower limit value for this total amount, it may, for example, be set to 1 mass% or more, 3 mass% or more, 6 mass% or more, 9 mass% or more, and the like.

The blended amount of the polar oil (component B) is not particularly limited as long as it is within a range satisfying the aforementioned conditions. However, it should preferably be 6 mass% or more for the purpose of imparting luster to the skin after application.

In the emulsion cosmetic of the present invention, it is preferable for the ratio [(B)/(a2 + a3 + a4)] of the blended amount of the polar oil (component B) to the total blended amount of the (a2) bis-ethyloxyphenol methoxyphenyl triazine, the (a3) diethylamino hydroxybenzoyl hexyl benzoate and the (a4) ethylhexyl triazone to be 0.6 or higher, since this further increases the stability, and it is more preferably 1.0 or higher. Though there is no restriction on the upper limit value for this ratio [(B)/(a2 + a3 + a4)], it is preferably approximately 6 or lower, more preferably approximately 5 or lower for the purpose of the usability of the cosmetic.

### (C) Higher alcohol

The higher alcohol (component C) used in the emulsion cosmetic of the present invention is not particularly limited as long as it is a higher alcohol having 6 or more carbon atoms that can be used in fields such as cosmetic products, pharmaceutical products and quasi-drug products, and includes saturated linear monohydric alcohols, unsaturated monohydric alcohols, and the like. Examples of saturated linear monohydric alcohols include dodecanol (lauryl alcohol), tridodecanol, tetradodecanol (myristyl alcohol), pentadecanol, hexadecanol (cetyl alcohol), heptadecanol, octadecanol (stearyl alcohol), nonadecanol, icosanol (arachyl alcohol), heneicosanol, docosanol (behenyl alcohol), tricosanol, tetracosanol (carnaubyl alcohol), pentacosanol, hexacosanol (ceryl alcohol), and the like. Examples of unsaturated monohydric alcohols include elaidyl alcohol and the like. In the present invention, saturated linear monohydric alcohols are preferable for the purpose of stability over time.

As the (C) higher alcohol, it is possible to use one or more of the above-mentioned types. In the present invention, it is preferable to use a mixture of two or more aliphatic alcohols, and mixtures of combinations such that the melting point of the mixture is 50 °C or higher are particularly preferred for the purpose of stability. For example, a combination of stearyl alcohol with behenyl alcohol can be described as a particularly preferable combination.

The blended amount of the (C) higher alcohol is preferably 0.1 to 10 mass%, more preferably 0.1 to 7 mass% relative to the total amount of the cosmetic. If the blended amount of the (C) higher alcohol is less than 0.1 mass% or more than 10 mass%, there are cases in which sufficient emulsion stability cannot be obtained.

### (D) Non-ionic surfactant

The non-ionic surfactant (component D) used in the oil-in-water emulsion cosmetic of the present invention is not particularly limited. Specific examples include polyethylene glycol fatty acid esters, polyoxyethylene glyceryl fatty acids, polyoxyethylene/methylpolysiloxane copolymers, polyoxyethylene sorbitan fatty acids, polyoxyethylene alkyl ethers, maltitol hydroxy aliphatic alkyl ethers, alkylated polysaccharides, alkylglucosides, sucrose fatty acid esters, polyoxyethylene glyceryl hydrogenated castor oil, polyoxyethylene sorbitan fatty acid esters, polyoxyethylene/polyoxypropylene block copolymers, tetrapolyoxyethylene/tetrapolyoxypropylene-ethylene diamine condensates, polyoxyethylene-beeswax/lanolin derivatives, alkanol amides, polyoxyethylene-propylene glycol fatty acid esters, polyoxyethylene-alkylamines, polyoxyethylene-fatty acid amides, alkylethoxydimethylamine oxides, trioleylphosphoric acid, and the like. In the emulsion cosmetic of the present invention, hydrophilic non-ionic surfactants having an HLB of 8 or higher such as, for example, beheneth-20, polysorbate 60 and PEG-40 stearate, can be named as particularly preferable examples. One or more types of non-ionic surfactants may be used.

The blended amount of the (D) non-ionic surfactant is preferably 0.1 to 20 mass%, more preferably 0.3 to 5 mass% relative to the total amount of the cosmetic. If the blended amount of the (D) non-ionic surfactant is less than 0.1 mass% or more than 20 mass%, then there are cases in which sufficient emulsion stability cannot be obtained.

### (E) Water

The cosmetic of the present invention is an oil-in-water emulsion, and thus contains water (component E). In the present invention, the aforementioned (C) higher alcohol forms, together with the (D) non-ionic surfactant and the (E) water, an aggregate (also known as an "a-gel") having a lamellar liquid-crystal structure. The blending ratio between the (C) higher alcohol and the (D) surfactant should preferably be within the range (C):(D) = 2:1 to 25:1 (molar ratio).

It is possible to blend other arbitrary components that can normally be blended into cosmetics or quasi-drugs, preferably having a sunscreen effect, into the emulsion cosmetic of the present invention, within a range not inhibiting the effects of the present invention.

The other arbitrary components are not limited, but may, for example, be powder components, colorants, humectants, oils (aside from the (A) oil-based ultraviolet ray absorbing agent and the (B) polar oil), thickeners, dispersing agents, preservatives, fragrances, various medicinal agents, and the like.

As the powder component, fine-particle or pigment-grade titanium oxide and zinc oxide can be named as representative examples. Other examples include metal oxide powders other than titanium oxide and zinc oxide, extender pigments such as talc, mica and kaolin, silica, and polymer powders such as silicone powder, polyethylene powder, poly methyl methacrylate powder and nylon powder, and the like. Since the emulsion cosmetic of the present invention becomes a sunscreen cosmetic having sufficient ultraviolet ray protection properties by combining specific ultraviolet ray absorbing agents (a1 to a4), it is not always necessary to blend in any ultraviolet ray scattering agents such as titanium oxide or zinc oxide. In other words, the emulsion cosmetic of the present invention includes embodiments not containing an ultraviolet ray scattering agent.

As colorants, it is possible to use those that are normally blended into makeup cosmetics, such as pigments and pearlescent pigments. Specifically, it is possible to blend one or a combination of two or more pigments selected from, but not limited to, inorganic white pigments (titanium dioxide and zinc oxide), inorganic red pigments (iron oxide (red iron oxide) and iron titanate), inorganic brown pigments (γ-ferric oxide), inorganic yellow pigments (yellow iron oxide and ocher), inorganic black pigments (black iron oxide, carbon and lower titanium oxides), inorganic violet pigments (mango violet and cobalt violet), inorganic green pigments (chromium oxide, chromium hydroxide and cobalt titanate), inorganic blue pigments (ultramarine blue and Prussian blue), pearlescent pigments (titanium oxide-coated mica, titanium oxide-coated bismuth oxychloride, titanium oxide-coated talc, colored titanium oxide-coated mica, bismuth oxychloride and argentine), metal powder pigments (aluminum powder and copper powder), organic pigments (Red No. 202, Red No. 205, Red No. 220, Red No. 228, Red No. 405, Orange No. 203, Orange No. 204, Yellow No. 205, Yellow No. 401 and Blue No. 404), zirconium, barium and aluminum lake organic pigments (Red No. 3, Red No. 104, Red No. 227, Red No. 401, Orange No. 205, Yellow No. 4, Yellow No. 202, Green No. 3 and Blue No. 1), natural pigments (chlorophyll, carotenoids (β-carotene), carthamin, cochineal, chalcone, curcumin, betanin, flavonols, flavones, anthocyanins, anthraquinones and naphthoquinones), functional pigments (boron nitride, photochromic pigments, synthetic fluorophlogopite, iron-containing synthetic fluorophlogopite and fine-particle composite powders (hybrid fine powders)), and the like. In the emulsion cosmetic of the present invention, these colorants may be blended by dispersion in either the internal oil phase or the external water phase.

Examples of humectants include polyhydric alcohols such as glycerin, 1,3-butylene glycol, dipropylene glycol and propylene glycol, and water-soluble polymers such as hyaluronic acid and chondroitin sulfate.

Examples of oils (aside from the (A) oil-based ultraviolet ray absorbing agent and the (B) polar oil) include volatile or non-volatile liquid oils such as hydrocarbon oils, ester oils, silicone oils and liquid oils/fats, and solid oils.

Though the production method is not particularly limited, the emulsion cosmetic of the present invention may be produced, for example, by preparing a melted oil part by melting the (C) higher alcohol, the (D) non-ionic surfactant, and the oils at a high temperature, adding the melted oil part to a heated water phase part containing (E) water and the other aqueous components, emulsifying the mixture by means of a conventional method, and cooling the emulsion.

The emulsion cosmetic of the present invention has a watery texture in use, which is characteristic of oil-in-water emulsions, and furthermore provides excellent ultraviolet ray protection effects by combining specific ultraviolet ray absorbing agents. Therefore, the emulsion cosmetic of the present invention can be favorably used in various formats, such as in cream form, milky lotion form and liquid form. The product form may be that of a skin-care cosmetic, including sunscreen cosmetics, or a makeup cosmetic, such as a makeup base or a foundation, having sunscreen effects.

### EXAMPLES

While the present invention will be explained in further detail by providing examples below, the present invention is not limited by these examples in any way. Where not otherwise noted, the blended amounts are indicated in mass% relative to the total amount of the composition in which those components are blended.

Oil-in-water emulsion sunscreen cosmetics were prepared by using the formulations indicated in Table 1 (milky lotions) and Table 2 (creams) below. The sunscreen cosmetics (samples) of each example were evaluated in accordance with the below-indicated criteria in the following categories (1) to (4).

### (1) Emulsion stability

### Evaluation criteria:

A: Precipitation and separation were not observed, and the emulsion was extremely stable.
B: Emulsification was possible, but precipitation was observed over time at low temperatures (0 °C).
C: Emulsification was possible, but enlargement of the emulsified particles was observed over time at high temperatures (37 °C).
D: Emulsification was possible, but separation was observed over time at room temperature.

### (2) Ultraviolet ray protection properties (light absorbance)

A sample of each example was applied to a PMMA substrate at a density of 1.75 mg/cm², and the light absorbance at a wavelength of 310 nm was measured by using a Hitachi U-4100 Spectrophotometer.

### (3) Feeling in use

Actual usage tests were performed by expert panelists, who evaluated the feeling in use in terms of the wateriness and the lack of stickiness, using the following criteria:
+: excellent
-: poor

### (4) Luster after application

Actual usage tests were performed by expert panelists, who evaluated the luster on the skin after application, using the following criteria:
A: excellent
B: normal
C: poor

**[Table 1]**

| | Ex. 1 | Ex. 2 | Ex. 3 |
|---|---|---|---|
| Phenylbenzimidazole sulfonic acid (a1) | 3 | 1 | 3 |
| bis-Ethylhexyloxyphenol methoxyphenyl triazine (a2) | 3 | 1 | 3 |
| Diethylamino hydroxybenzoyl hexyl benzoate (a3) | 2 | 1 | 5 |
| Ethylhexyl triazine (4) | 1 | 1 | 5 |
| Diisopropyl sebacate (B) | 15 | 6 | 26 |
| Stearyl alcohol | 1 | 1 | 1 |
| Behenyl alcohol | 1 | 1 | 1 |
| Beheneth-20 | 4 | 4 | 4 |
| Ion-exchanged water | 47.5 | 61.5 | 29.5 |
| Trisodium EDTA, 2H₂O | 0.1 | 0.1 | 0.1 |
| Xanthan gum | 0.2 | 0.2 | 0.2 |
| Succinoglycan | 0.2 | 0.2 | 0.2 |
| 1,3-butylene glycol | 5 | 5 | 5 |
| Glycerin | 5 | 5 | 5 |
| Dipropylene glycol | 5 | 5 | 5 |
| Triethanolamine | 2 | 2 | 2 |
| Ethanol | 5 | 5 | 5 |
| (a2) + (a3) + (a4) + (B) | 21 | 9 | 39 |
| (B)/(a2 + a3 + a4) | 2.5 | 2.0 | 2.0 |
| Emulsion stability | A | A | A |
| Absorbance | 1.011 | 0.613 | 1.374 |
| Feeling in use | + | + | + |

**[Table 2]**

| | Ex. 4 | Ex. 5 | Ex. 6 | Ex. 7 | Ex. 8 | Comp. Ex. 1 | Comp. Ex. 2 | Comp. Ex. 3 | Comp. Ex. 4 |
|---|---|---|---|---|---|---|---|---|---|
| Phenylbenzimidazole sulfonic acid (a1) | 3 | 3 | 3 | 3 | 3 | 3 | 3 | 3 | 3 |
| bis-Ethylhexyloxyphenol methoxyphenyl triazine (a2) | 3 | 3 | 3 | 3 | 3 | 3 | 5 | 3 | 3 |
| Diethylamino hydroxybenzoyl hexyl benzoate (a3) | 2 | 2 | 2 | 2 | 2 | 2 | 2 | 2 | 2 |
| Ethylhexyl triazine (4) | 1 | 1 | 1 | 1 | 1 | 1 | 1 | 1 | 1 |
| Diisopropyl sebacate (B) | 3 | 6 | 12 | 20 | 30 | 40 | 40 | 30 | 30 |
| Stearyl alcohol | 2 | 2 | 2 | 2 | 2 | 2 | 2 | - | - |
| Behenyl alcohol | 3 | 3 | 3 | 3 | 3 | 3 | 3 | - | - |
| Beheneth-20 | 1 | 1 | 1 | 1 | 1 | 1 | 1 | - | - |
| Ion-exchanged water | 69.9 | 66.9 | 60.9 | 52.9 | 42.9 | 32.9 | 30.9 | 48.7 | 48.5 |
| Trisodium EDTA, 2H₂O | 0.1 | 0.1 | 0.1 | 0.1 | 0.1 | 0.1 | 0.1 | 0.1 | 0.1 |
| (Acrylate/alkyl acrylate (C₁₀₋₃₀)) crosspolymer | - | - | - | - | - | - | - | 0.1 | 0.2 |
| Glycerin | 5 | 5 | 5 | 5 | 5 | 5 | 5 | 5 | 5 |
| Triethanolamine | 2 | 2 | 2 | 2 | 2 | 2 | 2 | 2.1 | 2.2 |
| Ethanol | 5 | 5 | 5 | 5 | 5 | 5 | 5 | 5 | 5 |
| a2 + a3 + a4 + B | 9 | 12 | 18 | 26 | 36 | 46 | 48 | 36 | 36 |
| (B)/(a2 + a3 + a4) | 0.5 | 1.0 | 2.0 | 3.3 | 5.0 | 6.6 | 5.0 | 5.0 | 5.0 |
| Emulsion stability | B | A | A | A | A | C | C | D | D |
| Feeling in use (immediately after preparation) | + | + | + | + | + | - | - | not measurable | not measurable |
| Luster after application | B | A | A | A | A | A | A | not measurable | not measurable |

As is clear from the results shown in Table 1 and Table 2, Examples 1-3 and 5-8, which satisfy all of the conditions recited in the claims of the present application, whether a milky lotion or a cream, had excellent stability, provided prominent ultraviolet ray protection properties, had a good feeling in use, and had excellent effects of imparting luster to the skin after application. However, in Example 4, in which the ratio (B)/(a2 + a3 + a4) was less than 0.6 and the blended amount of the polar oil was less than 6 mass%, there were no problems in terms of actual use and a stable emulsion state was maintained for a short period, but precipitation occurred over time at low temperatures, and the effect of imparting luster to the skin was normal, and though this was not a problem, prominent luster improvement was not observed.

In contrast therewith, in Comparative Examples 1 and 2, in which the blended amount of the oil-based components (a2 + a3 + a4 + B) exceeded 45 mass%, emulsification was possible, but the emulsion became unstable over time at high temperatures. Additionally, in the examples (Comparative Examples 3 and 4) not containing components (higher alcohols and non-ionic surfactants) forming an α-gel, in which emulsification was attempted by using polymer emulsifiers (acrylate/alkyl acrylate (C₁₀₋₃₀) crosspolymers) that are commonly used in oil-in-water emulsion cosmetics, the emulsion separated over time at room temperature and a stable emulsion was not able to be obtained.

Another formulation example of the emulsion cosmetic of the present invention will be provided below.

### Formulation Example 1:

| Blended Components | Blended amount (mass%) |
|---|---|
| Phenylbenzimidazole sulfonic acid (a1) | 3 |
| bis-Ethylhexyloxyphenol methoxyphenyl triazine (a2) | 0.3 |
| Diethylamino hydroxybenzoyl hexyl benzoate (a3) | 0.3 |
| Ethylhexyl triazine (a4) | 0.3 |
| Diisopropyl sebacate (B) | 5 |
| Stearyl alcohol | 2 |
| Behenyl alcohol | 3 |
| Beheneth-20 | 1 |
| Ion-exchanged water | balance |
| Trisodium EDTA, 2H₂O | 0.1 |
| Glycerin | 5 |
| Triethanolamine | 2 |
| Ethanol | 5 |
| Titanium dioxide | 0.5 |
| Iron oxide (red iron oxide) | 0.002 |
| Yellow iron oxide | 0.003 |

## Claims

1. An oil-in-water emulsion cosmetic **characterized by** containing:
(A) an ultraviolet ray absorbing agent containing
(a1) a sulfonic acid group-containing water-soluble ultraviolet ray absorbing agent,
(a2) bis-ethyloxyphenol methoxyphenyl triazine,
(a3) diethylamino hydroxybenzoyl hexyl benzoate, and
(a4) ethylhexyl triazone;
(B) a polar oil;
(C) a higher alcohol; and
(D) a non-ionic surfactant; wherein
a total blended amount of the (a2) bis-ethyloxyphenol methoxyphenyl triazine, the (a3) diethylamino hydroxybenzoyl hexyl benzoate, the (a4) ethylhexyl triazone, and the (B) polar oil is 45 mass% or less.

2. The emulsion cosmetic as in claim 1, wherein the ratio [(B)/(a2 + a3 + a4)] of the blended amount of the (B) polar oil to the total blended amount of the (a2) bis-ethyloxyphenol methoxyphenyl triazine, the (a3) diethylamino hydroxybenzoyl hexyl benzoate and the (a4) ethylhexyl triazone is 0.6 or higher.

3. The emulsion cosmetic as in claim 1, wherein the (A) ultraviolet ray absorbing agent consists only of the (a1) sulfonic acid group-containing water-soluble ultraviolet ray absorbing agent, the (a2) bis-ethyloxyphenol methoxyphenyl triazine, the (a3) diethylamino hydroxybenzoyl hexyl benzoate and the (a4) ethylhexyl triazone.

4. The emulsion cosmetic as in any one of claims 1 to 3, wherein the (B) polar oil comprises an ester oil having an IOB of 0.1 to 0.5.

5. The emulsion cosmetic as in any one of claims 1 to 4, wherein the (C) higher alcohol comprises a combination of stearyl alcohol and behenyl alcohol.

6. The emulsion cosmetic as in any one of claims 1 to 5, which is a sunscreen cosmetic.
